# EUROPÄISCHE PATENTSCHRIFT

(11) **EP 2 796 865 B1**
(45) Veröffentlichungstag und Bekanntmachung des Hinweises auf die Patenterteilung: **26.07.2017**
(21) Anmeldenummer: 14154262.1
(22) Anmeldetag: 07.02.2014
(51) Int. Cl.: G01N 27/22, G01N 33/44, B29B 7/74

(54) **Verfahren zur Identifizierung einer Gummimischung bei der Herstellung von Reifenbauteilen aus unvulkanisiertem Gummimaterial**
Method for identifying a rubber composition in the production of tyre components from unvulcanised rubber material
Procédé d'identification d'un mélange de caoutchouc lors de la fabrication de composants de pneumatiques à partir d'un matériau caoutchouc non vulcanisé

(30) Priorität: 25.04.2013 DE 102013104183
(43) Veröffentlichungstag der Anmeldung: 29.10.2014
(73) Patentinhaber: Continental Reifen Deutschland GmbH, 30165 Hannover (DE)
(72) Erfinder: Lacayo-Pineda, Jorge, 31535 Neustadt (DE); du Bois, Andre, 30657 Hannover (DE); Pavlinek, Vladimir, 76502 Otrokovice (CZ); Cermak, Roman, 76001 Zlin (CZ)
(74) Vertreter: Widjaja, Wira

(56) Entgegenhaltungen:
- EP-A2- 1 083 425
- DE-A1-102011 001 244
- A. H. SCOTT ET AL: "Effect of temperature and frequency on the dielectric constant, power factor, and conductivity of compounds of purified rubber and sulphur", JOURNAL OF RESEARCH, Bd. 11, 1. Januar 1934 (1934-01-01), Seiten 173-209, XP055130483,
- MARIANELLA HERNÁNDEZ ET AL: "Role of Vulcanizing Additives on the Segmental Dynamics of Natural Rubber", MACROMOLECULES, Bd. 45, Nr. 2, 24. Januar 2012 (2012-01-24), Seiten 1070-1075, XP055129983, ISSN: 0024-9297, DOI: 10.1021/ma202325k
- ONDREJ BOSÁK ET AL: "Electrical properties of a rubber blend used in the tyre industry", POLYMERS FOR ADVANCED TECHNOLOGIES, Bd. 18, Nr. 2, 16. November 2006 (2006-11-16), Seiten 141-143, XP055129849, ISSN: 1042-7147, DOI: 10.1002/pat.808

## Beschreibung

Die Erfindung betrifft ein Verfahren zur Identifizierung einer Gummimischung bei der Herstellung von Reifenbauteilen aus unvulkanisiertem Gummimaterial gemäß dem Oberbegriff des Anspruches 1.

Bei der Reifenherstellung werden spezielle Gummimischungen eingesetzt, die mit einem Mischer hergestellt werden und dann im Allgemeinen zwischengelagert werden. Die unterschiedlichen Gummimischungen werden anschließend mit Papierdatenblättern gekennzeichnet, damit sie anschließend der korrekten Weiterverarbeitung zugeführt werden können. Die vorkonfektionierten Laufstreifen für Fahrzeugreifen werden beispielsweise mit speziellen Extrudern hergestellt, wobei sichergestellt werden muss, dass der Extruder mit der korrekten Gummimischung bestückt wird. Es muss daher sichergestellt werden, dass dem Extruder immer die vorher spezifizierte Gummimischung zugeführt wird, damit der Laufstreifen beim fertigen Fahrzeugreifen die vorgegebenen Eigenschaften besitzt. Das gleiche gilt beim Kalanderprozess, mit dem ebenfalls unterschiedliche Reifenbauteile hergestellt werden.

Ein Verfahren der eingangs genannten Art ist aus der EP 1 083 425 A2 bekannt. In dieser Druckschrift ist offenbart, eine Materiallage aus einer Gummimischung online mit einer Messvorrichtung zu prüfen, wobei zwei auf unterschiedlichem elektrischen Potential befindliche Kontakte beidseitig der Gummilage angebracht werden und der elektrische Durchgangswiderstand zwischen diesen Stellen ermittelt und mit einem Referenzwert verglichen wird. Die DE 10 2011 001 244 A1 befasst sich mit einer Prüfmethode, bei welcher ein Probekörper des Gummimaterials in einer Messkammer eines Rheometers durch Kombination einer rheologischen Prüfmethode mit einer dielektrischen Prüfmethode geprüft wird. Dieses Verfahren gestattet keine online-Prüfung einer Materiallage aus einer Gummimischung.

Der Erfindung liegt die Aufgabe zugrunde, ein Verfahren zu schaffen, mit dem Gummimischungen auf einfache Weise identifiziert werden können, um sie nachfolgend einem bestimmten Herstellungsprozess für Reifenbauteile zuzuführen.

Gelöst wird die Aufgabe gemäß Anspruchs 1 mit einem Verfahren, bei dem die charakteristischen dielektrischen Materialeigenschaften der Gummimischung gemessen werden, die Messvorrichtung in Form einer zusammendrückbaren Messvorrichtung mit zwei gegenüberliegenden Elektroden ausgebildet ist, wobei die Materiallage der Gummimischung zwischen den Elektroden der Messvorrichtung eingeklemmt und an den Kontaktflächen zu der Gummimischung während der Messung planparallel und auf eine vorgegebene Materialdicke zusammen gedrückt wird.

Ein Vorteil der Erfindung ist insbesondere darin zu sehen, dass durch das Verfahren auf einfache Weise eine schnelle Identifizierung von Gummimischungen erfolgt, um sie nachfolgend einem bestimmten Herstellungsprozess für Reifenbauteile zuzuführen. Bei diesem Messverfahren erfolgt die Identifizierung der einzelnen Mischungen mit einer ausreichend hohen Genauigkeit. Dadurch kann eine versehentliche Verwechslung von Gummimischungen nahezu ausgeschlossen werden. Außerdem wird bei einer Identifizierung einer falschen oder stark verunreinigten Gummimischung direkt und automatisch ein Warnsignal ausgelöst. Dadurch wird sichergestellt, dass dem Extruder oder dem Kalander zu keinem Zeitpunkt eine falsche oder stark verunreinigte Gummimischung zugeführt wird.

Ein weiterer Vorteil des Verfahrens besteht darin, dass die Messung der dielektrischen Materialeigenschaften relativ einfach durchzuführen ist. Die in der Reifenherstellung eingesetzten Gummimischungen besitzen aufgrund unterschiedlicher Materialien spezifische dielektrische Materialeigenschaften.

Über die gemessene dielektrische Materialkonstante lassen sich vorher spezifizierte Gummimischungen eindeutig identifizieren und zuordnen.

Dadurch, dass die Messvorrichtung in Form einer zusammendrückbaren Vorrichtung mit zwei gegenüberliegenden Elektroden ausgebildet ist lässt sich eine Materiallage mit einer definierten Dicke zwischen den Elektroden realisieren und es wird eine hohe Messgenauigkeit zur Bestimmung der dielektrischen Materialeigenschaften erreicht.

Dadurch, dass die Materiallage zwischen den Elektroden der zusammendrückbaren Vorrichtung an den Kontaktflächen zu der Gummimischung während der Messung planparallel gedrückt wird, lassen sich die Messungen zur Bestimmung der dielektrischen Materialeigenschaften mit einer hohen Genauigkeit durchführen.

In einer weiteren vorteilhaften Weiterbildung der Erfindung ist vorgesehen, dass das Bereitstellen der Gummimischung mit unterschiedlichen Mischungsbestandteilen bei Schritt a) bei einem kontinuierlichen Kalander- oder Extrusionsprozess zur Herstellung von Reifenbauteilen erfolgt.

Auf diese Weise kann das Messverfahren automatisiert durchgeführt werden.

In einer weiteren vorteilhaften Weiterbildung der Erfindung ist vorgesehen, dass die Messvorrichtung direkt im Bereich für die Materialzuführung für den Extruder oder den Kalander positioniert ist.

Dadurch wird sichergestellt, dass dem Extrusionsprozess oder dem Kalanderprozess immer die korrekte Gummimischung zugeführt wird. Bei diesen Herstellungsprozessen ist im allgemeinen eine hohe Qualitätssicherung von Bedeutung.

In einer weiteren vorteilhaften Weiterbildung der Erfindung ist vorgesehen, dass die Messvorrichtung in Form eines Handgerätes ausgebildet ist, wobei mit dem Handgerät die auf Paletten bereitgestellten Gummimischungen kontrolliert werden können.

Dadurch lassen sich die auf Paletten bereitgestellten Gummimischungen auf einfache Weise kontrollieren.

In einer weiteren vorteilhaften Weiterbildung der Erfindung ist vorgesehen, dass die Messvorrichtung vor dem Eingangsbereich zu einem Mischer positioniert ist, wobei mit der Messvorrichtung das dem Mischer zugeführte Rohmaterial kontrolliert wird.

Das Messverfahren lässt sich ebenfalls bei der Mischungsherstellung auf einfache Weise einsetzen. Die dem Mischer zugeführten Rohmaterialien werden vorab mit dem Messvorrichtung kontrolliert, um diese anschließend dem Mischer zuzuführen.

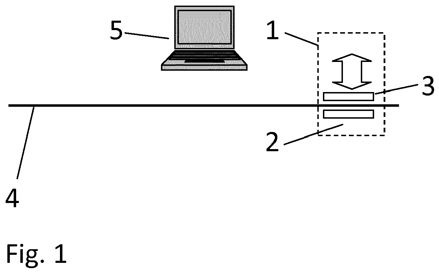

Anhand eines Ausführungsbeispiels soll die Erfindung näher erläutert werden. Es zeigt: Fig. 1: ein Ausführungsbeispiel für das Verfahren.

Bei diesem Ausführungsbeispiel ist die Messvorrichtung 1 z.B. in Form einer zusammendrückbaren Messvorrichtung ausgebildet. Die Gummimischung 4 ist in Form einer Materiallage ausgebildet, die beispielsweise auf Paletten vorgelagert wird. Der Bediener der Messvorrichtung ordnet ein Stück der Gummimischung 4 zwischen den beiden Elektroden 2 und 3 an. Diese werden mit auf einen vorgegebenen Abstand bzw. Materialdicke gegen die Gummimischung 4 gedrückt. Anschließend erfolgt die Messung der dielektrischen Materialeigenschaften der eingeklemmten Gummimischung 4.

Das Messgerät 5 ist bei diesem Ausführungsbeispiel außerhalb der Messvorrichtung 1 angeordnet. Nachdem die dielektrischen Materialeigenschaften beispielsweise in Form einer dielektrischen Materialkonstante von der Gummimischung 4 gemessen worden sind, erfolgt die Identifizierung der Gummimischung 4. Enthält beispielsweise die Gummimischung 4 starke Verunreinigung, dann wird mit der Messvorrichtung 1 ein abweichender Wert für die dielektrischen Materialeigenschaften der Gummimischung gemessen. Die entsprechende Gummimischung dürfte in diesem Fall nicht dem Extruder zur Herstellung von bestimmten Reifenbauteilen zugegeben werden. Ein ähnliches Szenario würde sich ergeben, wenn Gummimischungen am Extruder oder Kalander versehentlich vertauscht werden.

Über ein Warnsystem würde dann ein Warnsignal an der Messvorrichtung ausgegeben.

### Bezugszeichenliste (ist Teil der Beschreibung)

- 1: Messvorrichtung
- 2: Elektrode
- 3: Elektrode
- 4: Gummimischung in Form einer Materiallage
- 5: Messgerät

## Patentansprüche

1. Verfahren zur Identifizierung einer Gummimischung (4) bei der Herstellung von Reifenbauteilen aus unvulkanisiertem Gummimaterial mit folgenden Schritten:
a) Bereitstellen einer Gummimischung (4) in Form einer Materiallage mit unterschiedlichen Mischungsbestandteilen,
b) Positionierung einer Messvorrichtung (1) zur Bestimmung von Materialeigenschaften,
c) Messung von Materialeigenschaften der Gummimischung (4) mit einem an die Messvorrichtung (1) angeschlossenen Messgerät (5),
d) Empfangen und Auswerten der Messsignale mit dem Messgerät (5),
e) Identifizierung der Mischung durch einen Vergleich von vorab spezifizierten Materialeigenschaften mit der gemessenen Materialeigenschaft,
f) Aktivierung eines Warnsignales mit der Messvorrichtung (1),
wenn eine signifikante Abweichung zwischen den ermittelten und den vorgegebenen Materialeigenschaften identifiziert wird,
**dadurch gekennzeichnet, dass**
als Materialeigenschaften die charakteristischen dielektrischen Materialeigenschaften der Gummimischung (4) gemessen werden,
wobei die Messvorrichtung (1) in Form einer zusammendrückbaren Messvorrichtung mit zwei gegenüberliegenden Elektroden (2, 3) ausgebildet ist,
wobei die Materiallage der Gummimischung (4) zwischen den Elektroden (2, 3) der Messvorrichtung (1) in einem Bereich der Materiallage der Gummimischung (4) eingeklemmt und an den Kontaktflächen zu der Gummimischung während der Messung planparallel und auf eine vorgegebene Materialdicke zusammen gedrückt wird.

2. Verfahren nach einem der vorhergehenden Ansprüche,
**dadurch gekennzeichnet, dass**
das Bereitstellen der Gummimischung (4) mit unterschiedlichen Mischungsbestandteilen bei Schritt a) bei einem kontinuierlichen Kalander- oder Extrusionsprozess zur Herstellung von Reifenbauteilen erfolgt.

3. Verfahren nach einem der vorhergehenden Ansprüche,
**dadurch gekennzeichnet, dass**
die Messvorrichtung (1) direkt im Bereich für die Materialzuführung für den Extruder oder den Kalander positioniert ist.

4. Verfahren nach Anspruch 1,
**dadurch gekennzeichnet, dass**
die Messvorrichtung (1) in Form eines Handgerätes ausgebildet ist,
wobei mit dem Handgerät die auf Paletten bereitgestellten Gummimischungen (4) kontrolliert werden können.

5. Verfahren nach Anspruch 1,
**dadurch gekennzeichnet, dass**
die Messvorrichtung (1) vor dem Eingangsbereich zu einem Mischer positioniert ist,
wobei mit der Messvorrichtung (1) das dem Mischer zugeführte Rohmaterial kontrolliert wird.

## Claims

1. Method for identifying a rubber mixture (4) during the production of tyre components from non-vulcanized rubber material, having the following steps:
a) providing a rubber mixture (4) in the form of a material ply with different mixture constituents,
b) positioning a measurement device (1) for determining material properties,
c) measuring material properties of the rubber mixture (4) by means of a measurement unit (5) connected to the measurement device (1),
d) receiving and evaluating the measurement signals by means of the measurement unit (5),
e) identifying the mixture by way of a comparison of previously specified material properties with the measured material property,
f) activating a warning signal by means of the measurement device (1) if a significant deviation between the determined and the predefined material properties is identified,
**characterized in that**,
as material properties, the characteristic dielectric material properties of the rubber mixture (4) are measured,
wherein the measurement device (1) is formed in the manner of a measurement device with two opposite electrodes (2, 3) that can be pressed together,
wherein the material ply of the rubber mixture (4) is clamped between the electrodes (2, 3) of the measurement device (1) in a region of the material ply of the rubber mixture (4) and, at the contact surfaces with respect to the rubber mixture, is compressed in plane-parallel fashion and to a predefined material thickness during the measurement.

2. Method according to one of the preceding claims,
**characterized in that**
the provision of the rubber mixture (4) with different mixture constituents in step a) is performed in a continuous calendering or extrusion process for the production of tyre components.

3. Method according to one of the preceding claims,
**characterized in that**
the measurement device (1) is positioned directly in the region of the material feed for the extruder or the calender.

4. Method according to Claim 1,
**characterized in that**
the measurement device (1) is formed in the manner of a handheld unit,
wherein the rubber mixtures (4) provided on pallets can be inspected using the handheld unit.

5. Method according to Claim 1,
**characterized in that**
the measurement device (1) is positioned upstream of the inlet region into a mixer,
wherein the raw material fed to the mixer is inspected using the measurement device (1).

## Revendications

1. Procédé d'identification d'un mélange de caoutchouc (4) lors de la fabrication de composants de pneumatiques à partir d'un matériau de caoutchouc non vulcanisé, comprenant les étapes suivantes:
a) préparation d'un mélange de caoutchouc (4) sous la forme d'une couche de matériau avec différents ingrédients de mélange,
b) positionnement d'un dispositif de mesure (1) pour la détermination de propriétés de matériau,
c) mesure de propriétés de matériau du mélange de caoutchouc (4) avec un appareil de mesure (5) raccordé au dispositif de mesure (1),
d) réception et exploitation des signaux de mesure avec l'appareil de mesure (5),
e) identification du mélange par une comparaison de propriétés de matériau spécifiées au préalable avec les propriétés de matériau mesurées,
f) activation d'un signal d'avertissement avec le dispositif de mesure (1), lorsqu'un écart significatif est identifié entre les propriétés de matériau déterminées et prédéterminées,
**caractérisé en ce que** l'on mesure comme propriétés de matériau les propriétés de matériau diélectriques caractéristiques du mélange de caoutchouc (4),
dans lequel le dispositif de mesure (1) est configuré sous la forme d'un dispositif de mesure compressible avec deux électrodes opposées (2, 3),
dans lequel on pince la couche de matériau du mélange de caoutchouc (4) entre les électrodes (2, 3) du dispositif de mesure (1) dans une région de la couche de matériau du mélange de caoutchouc (4) et on la comprime sur les faces de contact avec le mélange de caoutchouc pendant la mesure en position plane parallèle et jusqu'à une épaisseur de matériau prédéterminée.

2. Procédé selon la revendication précédente, **caractérisé en ce que** l'on effectue la préparation du mélange de caoutchouc (4) avec différents ingrédients de mélange à l'étape a) au cours d'un procédé continu de calandrage ou d'extrusion pour la préparation de composants de pneumatiques.

3. Procédé selon l'une quelconque des revendications précédentes, **caractérisé en ce que** le dispositif de mesure (1) est positionné directement dans la région de l'alimentation de matériau pour l'extrudeuse ou la calandre.

4. Procédé selon la revendication 1, **caractérisé en ce que** le dispositif de mesure (1) est réalisé sous la forme d'un appareil portatif, dans lequel les mélanges de caoutchouc (4) préparés sur des palettes peuvent être contrôlés avec l'appareil portatif.

5. Procédé selon la revendication 1, **caractérisé en ce que** le dispositif de mesure (1) est positionné devant la région d'entrée d'un mélangeur, dans lequel on contrôle avec le dispositif de mesure (1) le matériau brut fourni au mélangeur.
